# EUROPEAN PATENT SPECIFICATION

(11) **EP 2 473 117 B1**
(45) Date of publication and mention of the grant of the patent: **17.08.2016**
(21) Application number: 10814273.8
(22) Date of filing: 25.08.2010
(51) Int. Cl.: A61B 17/03, A61B 17/04, A61B 17/24, A61L 31/14, A61L 31/10

(54) **METHODS AND SYSTEMS FOR TISSUE FASTENING**
VERFAHREN UND SYSTEME ZUR GEWEBEBEFESTIGUNG
PROCÉDÉS ET SYSTÈMES DE FIXATION DE TISSUS

(30) Priority: 03.09.2009 US 239677 P
(43) Date of publication of application: 11.07.2012
(73) Proprietor: MimOSA Medical, Inc., Menlo Park, CA 94025 (US)
(72) Inventor: GONZALES, Donald, A., San Antonio, TX 78249 (US); NIEDERAUER, Gabriele, G., San Antonio, TX 78249 (US); OHASHI, Kevin, San Antonio, TX 78249 (US); NATALE, Anthony, San Antonio, TX 78249 (US); DINGER, Fred, B., III, San Antonio, TX 78249 (US)
(74) Representative: Patent Boutique LLP
(86) International application number: PCT/US2010/046592
(87) International publication number: WO 2011/028564

(56) References cited:
- WO-A1-99/42147
- US-A- 5 222 976
- US-A- 5 366 459
- US-A1- 2002 010 490
- US-A1- 2004 176 783
- US-A1- 2005 288 710
- US-A1- 2006 167 480
- US-A1- 2008 208 265
- US-A1- 2008 215 090

## Description

### Background Information

There are over 750,000 tonsillectomies performed each year in the U.S., and 1.5 million performed worldwide. The most common complications of tonsillectomy are pain and bleeding. Post-operative pain frequently leads to hospitalization or at least a visit to a physician due to the pain *per se* and/or dehydration caused by decreased oral intake due to the pain. Post-operative bleeding also occurs in approximately 5% of all cases. The occurrence of bleeding is bimodal, and bleeding is frequently observed at post-operative day 1 and post-operative day 5-9. Although, historically, tonsillectomy was primarily performed to treat recurrent infection (tonsillitis), 77% of tonsillectomies are now performed to treat sleep-disordered breathing (SDB) Friedman et al., Otolaryngol. Head and Neck Surg. 140(6):800-8. Sleep disordered breathing is a form of obstructive sleep apnea (OSA) in the pediatric population. Although tonsillectomy alone has shown some success in treating SDB, studies have found additional measures need to be performed to treat a growing number of patients, specifically closure of the tonsillar pillars after the removal of the tonsils. There is evidence that closing the tonsillar pillars (e.g., via suturing) improves sleep apnea scores after tonsillectomy, leads to better healing, and reduces post-operative pain. Nandapalan et al., Clin. Otolaryngol. Allied Sci. 20(2):127-29, April 1995; Gene et al., Int. J. Pediatr. Otorhinolaryngol. 70(4):725-30, April 2006;Guilleminault et al. Laryngoscope. 114 (1): 132-7. Furthermore, closing of the tonsillar pillars is currently performed in conjunction with uvulopalatopharyngoplasty (UPPP) to treat adults with sleep apnea. Although closing of the tonsillar pillars has been shown to be advantageous, tonsillar pillar dehiscence is a problem after UPPP with tonsillectomy. The overall incidence of dehiscence is approximately 40% and has been found to be independent of the dissection method (*i.e*., cold scalpel versus electocautery). Altman et al., Laryngoscope 114(2):294-6, Feb. 2004; Because of this high rate of dehiscence and the amount of surgical time needed to close the tonsillar pillars via suturing, it is not recommended in all tonsillectomies.

Furthermore, mucosal suturing dehiscence is not isolated to the tonsillar pillars, but is seen throughout the digestive tract, specifically closures under tension. High dehiscence rates are seen in closure of oral, pharyngeal, laryngeal, esophageal, and intestinal tissue.

Surgical sealants such as fibrin-based glues have also been used in tonsillectomy as an effective substitute for electrocautery. Sealant use has been shown to provide effective hemostasis and sealing. Vaiman et al., Ann. Otol. Rhinol Laryngol. 112(5):410-14, May 2003. However, sealant does not remain adherent for the amount of time necessary for the tissue to heal. US patent No 2008/208265 A1 discloses a suture fastening device which forms the basis of the preamble of claim 1.

### Summary

Embodiments of the present invention may be used to address a need in the art for an effective system for closing mucosa, particularly mucosa of the oral cavity, oronasopharynx, hypopharynx, laryngeal, esophageal and intestinal surfaces. Such a system preferably provides for the effective closure of mucosal tissues without dehiscence. The system may be used in surgical procedures of the oronasopharynx (e.g., UPPP, tonsillectomy, uvulopalatal flap (UPF) technique, dental procedures, laryngectomy, pharyngectomy, esophagectomy, tumor removal, *etc.).* Embodiments of the present invention provide such devices for the effective closure of mucosa as well as instruments for the delivery of these novel fastening devices. Embodiments of the present invention save time and can lead to better outcomes than current suturing techniques. For example, embodiments of the invention may reduce dehiscence rates, reduce bleeding, reduce pain, speed healing, reduce surgical time, and/or improve sleep apnea score results.

In one aspect, the invention provides fastening devices for closing tissues, particular mucosal tissues. Mucosa is typically difficult to suture given its propensity to tear. Any tension may lead to tearing of the suture material through the mucosa secondary to the cross-sectional round geometry of the suture. Because of the round shape, the tension on the suture is concentrated in a minimal area. The inventive fastening device is designed to prevent the tearing of mucosa by the fastening device. In certain embodiments, the device may include flat, elongated, or rectangular cross-sectional shapes of the fastening device to avoid or minimize tearing that is frequently seen with round suture material. The thickness of the material used to construct the closing device may range from approximately 0.5-2 mm. As will be appreciated by one of skill in the art, the fastening devices may come in a variety of sizes for use in different applications or different subjects. For example, smaller sized fastening devices are preferable for use in the pediatric population.

The device may be either a one piece or a two-piece system. In certain embodiments, the device is a molded, one piece fastening device. The device may be made of a resorbable or non-resorbable material (*e.g*., a polymeric material). In certain embodiments, the device is made of a resorbable polymeric material such as a polyester. In certain embodiments, the device is made of a resorbable material such as poly(lactic-co-glycolic acid).

The device may be used anywhere in the body of a subject; however, it is particularly useful in the oral cavity, oronasopharynx, and hypopharynx of the head and neck. In certain embodiments, the device is particularly suited for closing the tonsillar pillars in a uvulopalatalpharyngoplasty (UPPP) and/or tonsillectomy. In certain embodiments, the device is particularly suited for use in a uvulopalatal flap (UPF) procedure. In certain embodiments, the device is suited for use in a dental procedure such as tooth extraction. The device may also be used in surgeries involving the gastrointestinal and genitourinary systems.

The inventive fastening devices may be optionally coated. The coating may include a timed release formulation of a pharmaceutical agent such as an anti-inflammatory agent, a steroid, antibiotic, anesthetics, pain reliever, hemostatic agent, *etc.* The device may also be coated to make the device more biocompatible. Many coatings for medical devices are known in the art and may be applied to the inventive fastening devices.

Exemplary provides methods of using the fastening devices and/or the instruments are described. The devices and instruments may be used in a variety of surgeries or procedures. The surgeries or procedures may involve the approximation of mucosal surfaces of the head and neck. The surgeries or procedures may involve the oral cavity, oropharynx, hypopharynx, throat, or laryngeal surfaces. The surgery or procedure is one typically performed by a certified physician or other health care professional. In certain cases the surgery is a UPPP. In other cases, the surgery is a tonsillectomy. An exemplary method of closing the tonsillar pillars using the inventive fastening devices and apparatuses is described. A sealant, such as a fibrin based product, chitosan based product, thrombin based products, alpha-cellulose based products, collagen based products, albumin based products can be used in conjunction with the fastening devices in order to reduce pain, reduce bleeding, and/or otherwise improve the outcome. A coating, such as protein or growth factor based products, can be used in conjunction with the fastening devices in order to enhance healing and/or otherwise improve the outcome. If the surgery is a tonsillectomy the sealant may be placed on the tonsillar bed. The surgery may be a UPF procedure. The surgery may be a removal of a tumor of the head or neck. The surgery may be a dental or oral surgery. The surgery may involve the closing of laryngeal or pharyngeal defect. The devices and instruments may also be used in other areas of the body including the gastrointestinal tract and genitourinary system. The devices and instruments may also be used in neurosurgery such as dural closure.

One of the many advantages of the inventive system is that it offers physicians an alternative to suturing, which has the problem of a high rate of dehiscence, bleeding, pain, and other complications. The inventive system allows for the efficient closure of mucosa, particularly in the oronasopharynx, the oral cavity, oropharynx, hypopharynx, and laryngeal surfaces. Overall, the inventive system can reduce surgical time, thereby reducing the time a subject is under anesthesia, and improves surgical outcomes.

In certain embodiments, the device is made of a biocompatible material. In specific embodiments, the device is made of a biocompatible polymer. In specific embodiments, the device is made of a biocompatible, bioresorbable material. In certain embodiments, the biocompatible, bioresorbable material is a polyester, a polyanhydride, a polyphosphazene, a polyacrylate, or a polymethacrylate. In certain embodiments, the device is made of poly(lactic-co-glycolic acid) (PLGA). In specific embodiments, the device is made of poly(L-lactic-co-glycolic acid) (90% glycolide:10% L-lactide). In specific embodiments, the biocompatible, bioresorbable material is absorbed in approximately 1-4 weeks during use. In specific embodiments, the biocompatible, bioresorbable material is absorbed in approximately 4-6 weeks during use. In other embodiments, the biocompatible, bioresorbable material is absorbed in approximately 6-8 weeks during use. In other embodiments, the biocompatible, bioresorbable material is absorbed in approximately 2-3 months during use.

In certain embodiments, the device is made of a non-biodegradable material. In certain embodiments, the device is coated. In specific embodiments, the device is coated with Teflon (PTFE). In other embodiments, the device is coated with hyaluronidate. In certain embodiments, the device is coated with a polymer, and in specific embodiments the coating comprises a pharmaceutical agent. In specific embodiments, the pharmaceutical agent is selected from the group consisting of hemostatic agents, anesthetics, pain relievers, anti-inflammatory agents, and antibiotics.

The inventive fastening devices for use in approximating mucosa or other tissues are designed to reduce or prevent tearing of the tissue or other damage to the tissue. Although suited for use in closing or approximating mucosal tissues, the fastening devices may be used in other non-mucosal tissues. Embodiments of the device may also be used to approximate a mucosal surface to a non-mucosal surface; to approximate a mucosal surface to non-mucosal surface; or to approximate a non-mucosal surface to a non-mucosal surface. Embodiments of the fastening device may be of a variety of shapes and sizes. In order to minimize the tearing of mucosa by the fastening device, certain embodiments do not include a round cross-sectional area. Instead, the cross-section of certain embodiments of the device is flattened, oval, polygonal, rectangular, or square. In particular embodiments, the cross-section of the part of the device penetrating the tissue is flattened, oval, polygonal, rectangular, or square. Such a cross-sectional shape reduces the likelihood of the device from tearing through the tissue which has been closed. The device may be molded from a biocompatible, bioresorbable polymeric material.

The thickness of the device may also determined by the use. The thickness of the device can be important in reducing or preventing the tearing of tissue by the device. Thicker devices are typically less prone to tear tissue held by the device. In certain embodiments, the thickness of the device is typically approximately 0.2 mm to approximately 2 mm. In certain embodiments, the thickness or height is approximately 0.5 mm to approximately 2 mm. In certain embodiments, the height is approximately 0.5 mm to approximately 1.5 mm. In certain embodiments, the height is approximately 0.5 mm, approximately 0.6 mm, approximately 0.7 mm, approximately 0.8 mm, approximately 0.9 mm, approximately 1.0 mm, approximately 1.1 mm, approximately 1.2 mm, approximately 1.3 mm, approximately 1.4 mm, approximately 1.5 mm, approximately 1.6 mm, approximately 1.7 mm, approximately 1.8 mm, approximately 1.9 mm, or approximately 2.0 mm. Devices thicker or thinner than the above recited ranges are also considered to be part of the present invention.

The fastening device may be constructed of any biocompatible material. In certain embodiments, the material is rigid enough to allow an end of the device (e.g., the male end) to pierce mucosa. The material may be a natural or non-natural material. The material may be bioresorbable or non-bioresorbable. The material may be polymeric. In certain embodiments, the fastening device is made of a bioresorbable polymeric material. In certain embodiments, the fastening device is made of a bioresorbable, synthetic polymeric material. In certain embodiments, the polymer is a co-polymer. In certain embodiments, the polymer is block polymer. In certain embodiments, the polymer is linear polymer. In certain other embodiments, the polymer is a branched polymer. In certain embodiments, the polymer is a dendritic polymer. In certain embodiments, the polymer is a cross-linked polymer. In certain embodiments, the polymer is a polyester, polyurethane, polyvinyl chloride, polyethylene, polyolefin, polyanhydride, polyamide, polycarbonates, polycarbamate, polyacrylate, polymethacrylate, polystyrene, polyurea, polyether, polyalkylether, or polyamine. Exemplary polymers that may be used to make the device include poly(lactic acid), poly(glycolic acid), poly(lactic-co-glycolic acid) (PLGA), poly(anhydride), polyphosphazenes, and poly(caprolactone). In certain embodiments, the polymer is a poly(glycolide-co-lactide) (PLGA). In certain embodiments, the device is made of 50% D,L-lactide and 50% glycolide co-polymer. In certain embodiments, the device is made of 50% L-lactide and 50% glycolide co-polymer. In certain embodiments, the device is made of 85% D,L-lactide and 15% glycolide co-polymer. In certain embodiments, the device is made of 85% L-lactide and 15% glycolide co-polymer. In certain embodiments, the device is made of 90% D,L-lactide and 10% glycolide co-polymer. In certain embodiments, the device is made of 90% L-lactide and 10% glycolide co-polymer. In certain embodiments, the polymer is polyglycolic acid. In certain embodiments, the polymer is poly-β-hydroxybutyrate. In certain embodiments, the polymer is polyacrylic acid ester. In certain embodiments, the device is made of Pebax, Polyimide, Braided Polyimide, Nylon, PVC, Hytrel, HDPE, or PEEK. In certain embodiments, the device is made of a fluorinated polymer such as PTFE, PFA, FEP, and EPTFE. In certain embodiments, the device is made of latex. In certain embodiments, the device is made of a natural polymer. In certain embodiments, the natural polymer is a polysaccharide such as cellulose or derivatives thereof. In certain embodiments, the natural polymer is a protein. The fastening device may be made of a material that is bioabsorbed after the device is no longer needed (e.g., after the tissues have healed). For example, the device may degrade *in vivo* after 1 week, 2 weeks, 3 weeks, 1 month, 2 months, 3 months, 4 months, 5 months, 6 months, *etc.* In certain embodiments, the device is designed to degrade after approximately 4-6 weeks *in vivo.*

In certain embodiments, the fastening device may also be constructed of a shape memory plastic or metallic material. Such shape memory plastic or metallic materials can return to some previously defined shape or size when subjected to the appropriate thermal procedure or similar form of energy. That is, shape memory alloys or plastics can be plastically deformed at some relatively low temperature and, upon exposure to some higher temperature, will return to their original shape. Metal materials that exhibit the shape memory effect include a number of copper allow systems and the alloys of gold-cadmium, nickel-aluminum, and iron-platinum. In addition, plastic materials that exhibit the shape memory effect have been developed and include, for example, materials disclosed in U.S. Patent Nrs. 6,720,402, 6,388,043, 6,160,084. In certain embodiments, the fastening device may be provided in a shape that is easily inserted into the mucosa.Thermal energy may then be applied to cause the fastening device to change shape (e.g., make the fastening device close together), thus providing a rapid closure of the fastener through the mucosal flaps with merely an application of heat or energy.

In certain embodiments, the fastening device may comprise an elastomeric material, including for example, a polyhydroxyalkanoate (PHA) material. In certain embodiments, the fastening device may comprise a crystalline or amorphous polymer combined with an elastomeric polymer. For example, a highly crystalline polylactide may be blended with a polyhydroxybutarate; specifically 80-97% Poly-L-Lactides (PLLA) and 20-3% PHA in certain embodiments. Similarly, caprolactone or trimethyl carbonate may be added to a crystalline polymer to make it more elastic. Elasticity of the construct can be achieved through the addition of the caprolactone or trimethyl carbonate to a lactide or glycolide monomer since the caprolactone and trimethyl carbonate have relatively low melting temperatures (e.g., 60°C for carpolactone).

Such material can provide an elasticity that is similar to that of the surrounding tissue. By providing a similar elasticity to the surrounding material, the fastening device can flex with the surrounding tissue. This can increase the ability of the fastening device to maintain approximation of the tissue during healing, and can reduce the likelihood that the fastening device will tear or otherwise damage the surrounding tissue.

In certain embodiments, the fastening device is coated. The coating may be biocompatible, and in certain embodiments, the coating is a polymeric coating. The coating may provide the release of a pharmaceutical agent. The agent may be released over hours, to days, to weeks, to months. In certain embodiments, the coating is a polymeric coating impregnated with a therapeutic agent. Classes of therapeutic agents that may be delivered by the device include DNA, RNA, nucleic acids, proteins, peptides, or small molecules. Exemplary therapeutic agents include antibiotics, anti-inflammatory agents, corticosteroids, vasoconstrictors, vasodilators, coagulants, pain relievers, *etc.* In other embodiments, the coating is Teflon. The device may be coated with a polysaccharide such as hyaluronate. The coating may also include a radioopaque agent for imaging of the device. The coating may also be designed to make the fastening device more biocompatible.

Embodiments of the inventive system have a wide variety of uses in medicine. In certain embodiments the system is particularly useful for closing in a surgical procedure. The devices may be used on any tissue in the body of any animal. In certain embodiments, the subject is a mammal. In certain particular embodiments, the subject is a human. As discussed, the system is particularly useful for closing or approximating mucosal tissues such as those found in the oral cavity, nasopharynx, hypopharynx, nasal cavity, pharynx, larynx, or esophagus. The system may also be used in the gastrointestinal or genitourinary system. Exemplary procedures that may utilize the inventive devices include UPPP, tonsillectomy, UPF procedure, tumor removal, dental extraction, dental procedure, total or partial laryngectomy, esophagectomy, pharyngectomy, *etc.* The device may be used to approximate two tissues, passing the device through the tissues, and fastening the device closed. These steps may be aided by the instruments described herein. In certain embodiments, the fastening devices are used with a sealant such as a natural or synthetic sealant (e.g., fibrin-based sealant, collagen-based sealant, fibrin-based sealant).

In certain embodiments, the device is made of a biocompatible, bioresorbable material. The device may be coated with a protein or growth factor-based product to enhance healing of the tissue during use.

Certain embodiments include a fastening device for fastening tissue, where the fastening device may comprise: a first suture comprising a first fastening member; a second suture comprising a second fastening member; and a coupling member coupling the first suture to the second suture, where the coupling member is configured to slide relative to the first and second suture. In specific embodiments, the coupling member may comprise a first aperture and a second aperture, and the first suture may extend through the first aperture and the second suture may extend through the second aperture.

In particular embodiments, the first fastening member may be coupled to a first end of the first suture and the second fastening member may be coupled to a first end of the second suture. In certain embodiments, the first fastening member and the second fastening member may each comprise a pointed end configured to penetrate tissue.

Specific exemplary methods include a method of fastening tissue, where the exemplary method may comprise: providing a fastening device according to an exemplary embodiment described herein; approximating a first portion of tissue to a second portion of tissue; piercing the first portion of tissue with the first fastening member; piercing the second portion of tissue with the second fastening member; and moving the coupling member toward the first and second fastening members. In particular, the method may comprise: tying a first knot in the first suture so that the coupling member is positioned between the first knot and the first fastening member; and tying a second knot in the second suture so that the coupling member is positioned between the second knot and the second fastening member. In certain examples, the method may comprise: tying the first suture and the second suture in a knot so that the coupling member is positioned between the knot and the first and second fastening members.

Specific exemplary methods include a method of fastening tissue, where the exemplary method may comprise: providing a fastening device according to an exemplary embodiment described herein; approximating a first portion of tissue to a second portion of tissue; inserting the first penetrating member into the first portion of tissue; and inserting the second penetrating member into the second portion of tissue. In particular embodiments, the fastening device may comprise an elastomeric material. In certain embodiments, the elastomeric material may be a polyhydroxyalkanoate material. In specific embodiments, the elastomeric material may be combined with a crystalline or amorphous polymer. In particular embodiments, the fastening device may have an elasticity equivalent to that of mucosal tissue. In certain embodiments, the fastening device may comprise a shape memory material.

### Brief Description of the Drawings

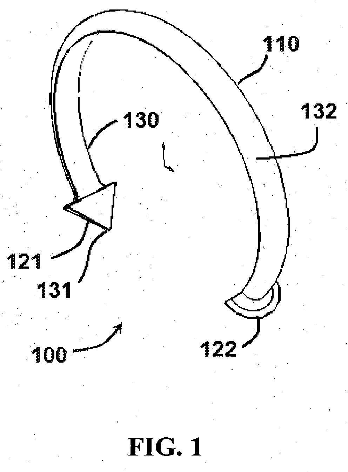

FIG. 1 illustrates a perspective view of an exemplary fastening device not forming part of the present disclosure.

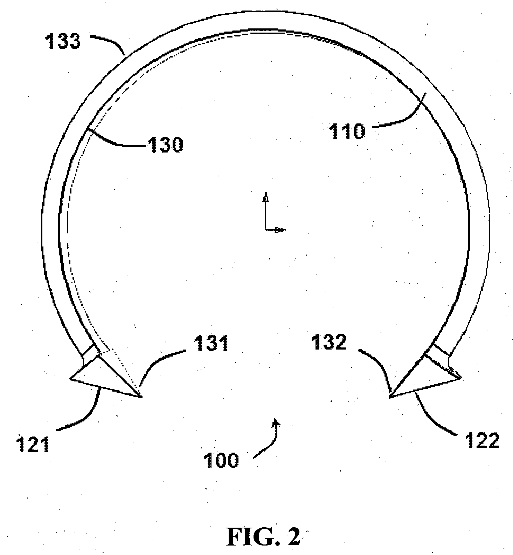

FIG. 2 illustrates a front view of the embodiment of FIG. 1.

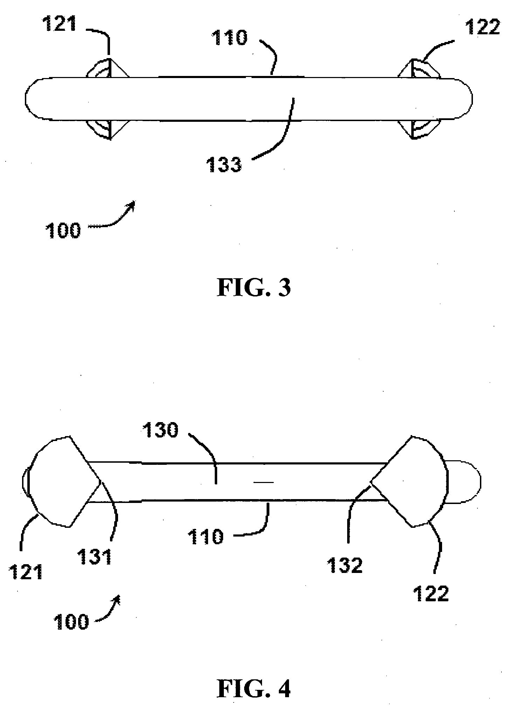

FIG. 3 illustrates a top view of the embodiment of FIG. 1.
FIG. 4 illustrates a bottom view of the embodiment of FIG. 1.

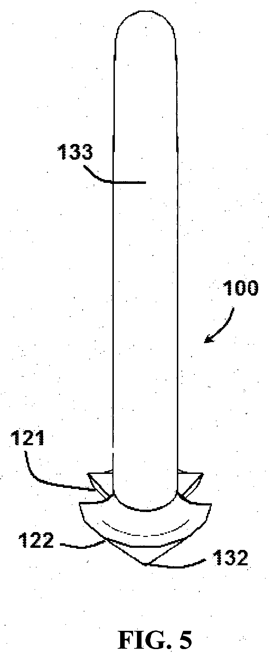

FIG. 5 illustrates a side view of the embodiment of FIG. 1.

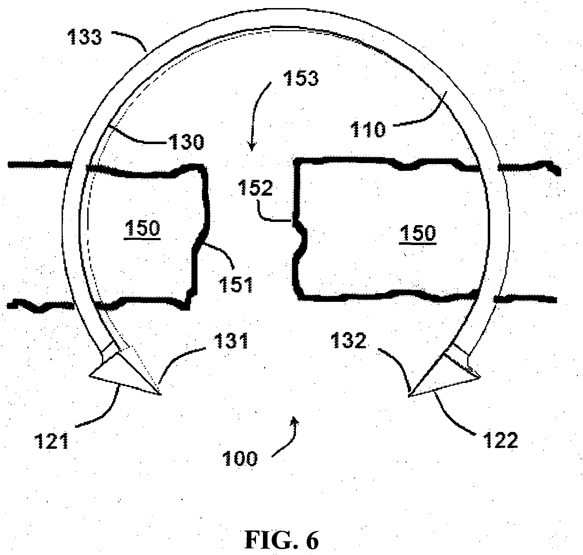

FIG. 6 illustrates a partial cross-section view of FIG. 1 during use.

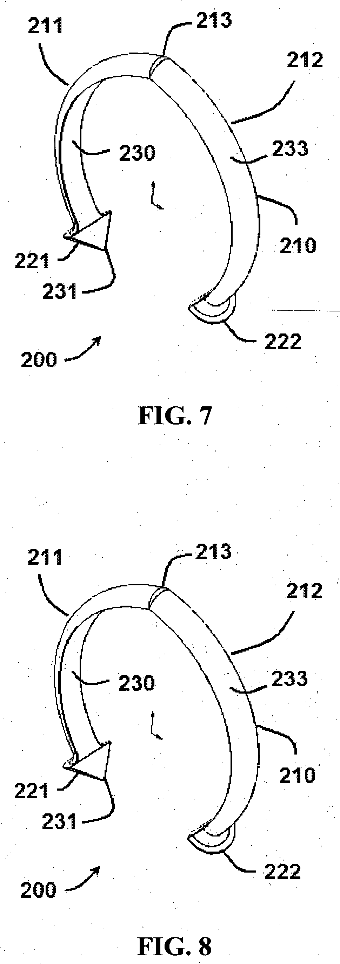

FIG. 7 illustrates a perspective view of a fastening device to
FIG. 8 illustrates a front view of the embodiment of FIG. 1.

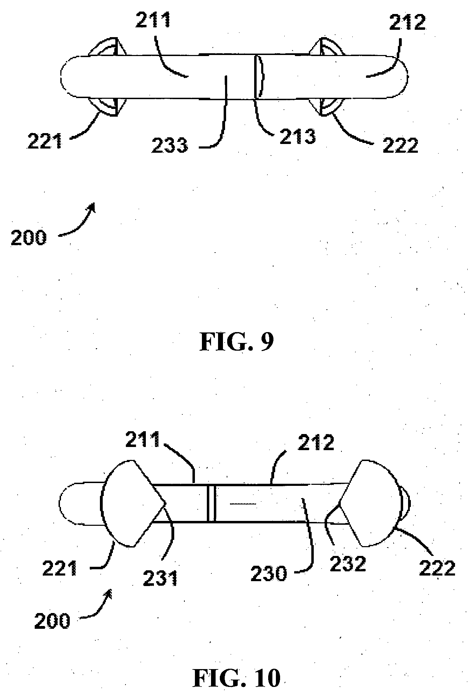

FIG. 9 illustrates a top view of the embodiment of FIG. 1.
FIG. 10 illustrates a bottom view of the embodiment of FIG. 1.

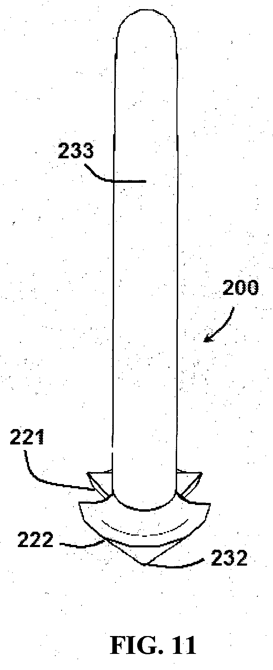

FIG. 11 illustrates a side view of the embodiment of FIG. 1.

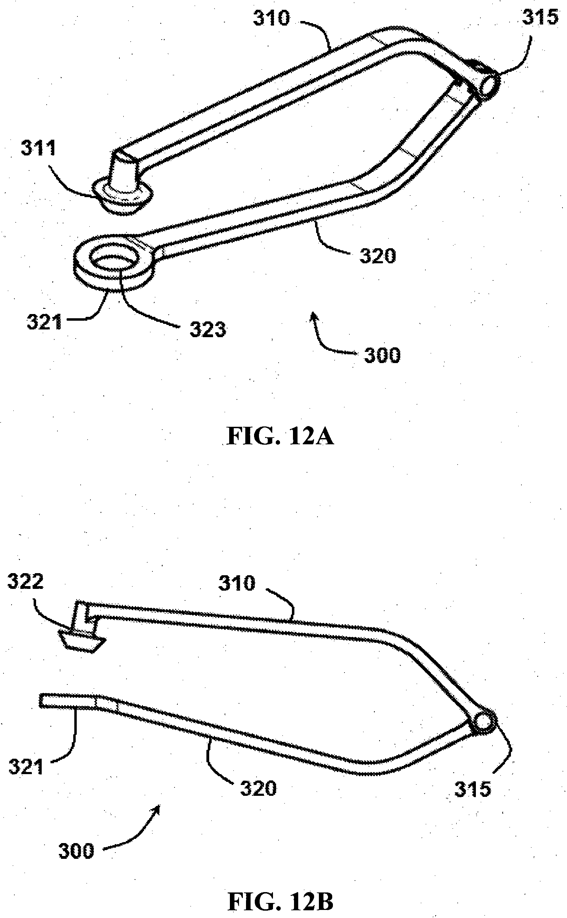

FIG. 12A illustrates a perspective view of a fastening device.
FIG. 12B illustrates a side view of FIG. 12A.

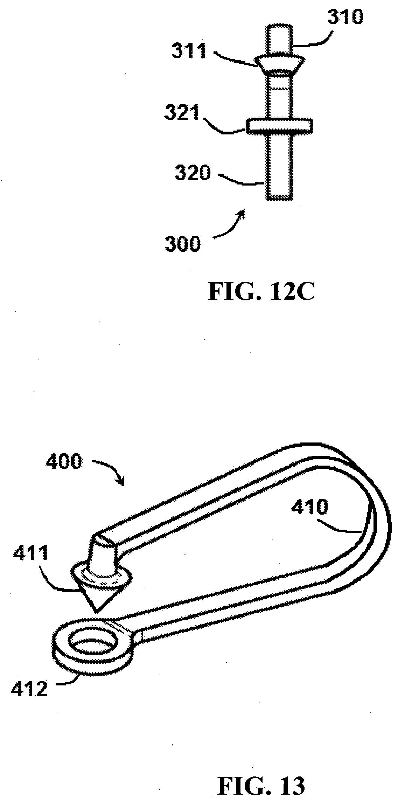

FIG. 12C illustrates an end view of FIG. 12A.
FIG. 13 illustrates a perspective view of an exemplary fastening device not forming part of the present disclosure.

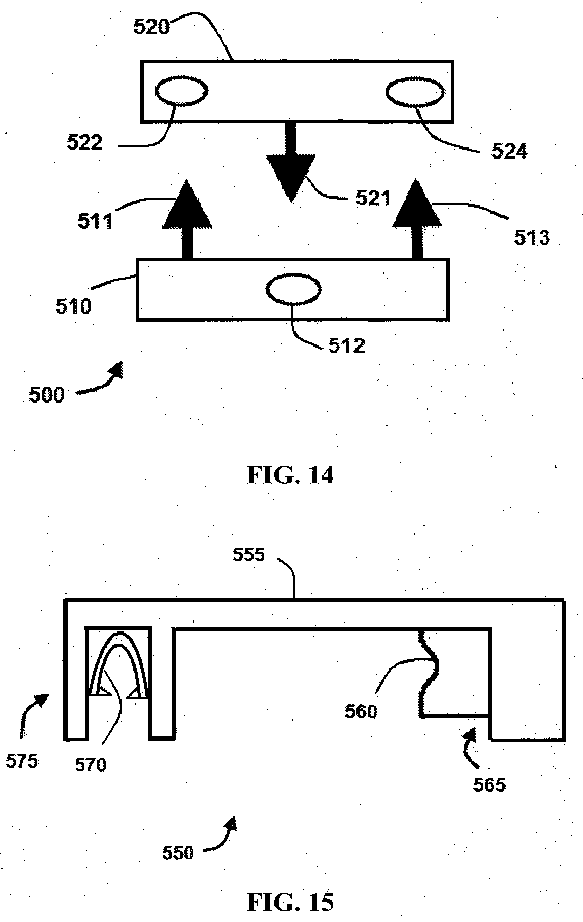

FIG. 14 illustrates a perspective view of an exemplary fastening device not forming part of the present disclosure.
FIG. 15 illustrates an instrument configured to place an exemplary fastening device not forming part of the present disclosure.

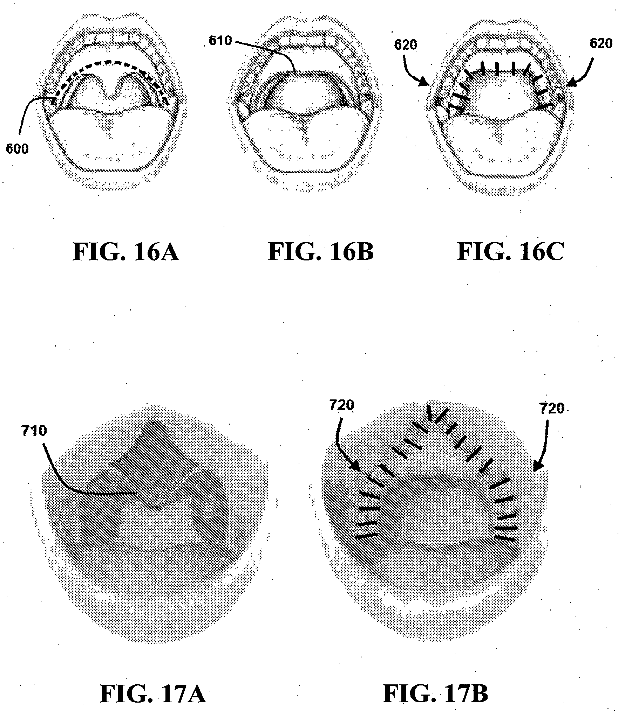

FIGS. 16A-16C illustrate an exemplary surgical procedure utilizing fastening devices.
FIGS. 17A-17B illustrate an exemplary surgical procedure utilizing fastening devices.

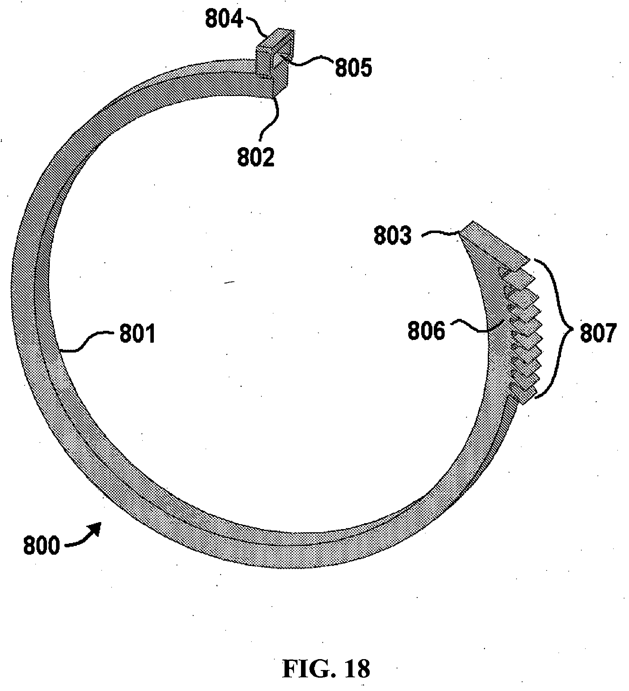

FIG. 18 illustrates a perspective view of an exemplary fastening device not forming part of the present disclosure.
FIG. 19 illustrates a perspective view of a fastening device according to exemplary embodiments of the present disclosure.

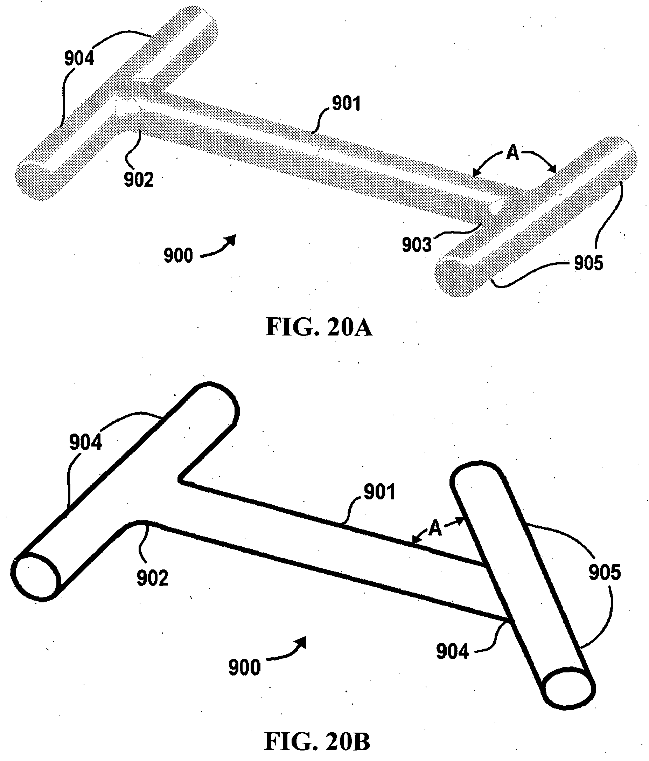

FIG. 20A illustrates a perspective view of an exemplary fastening device in a first position, not forming part of the present disclosure.
FIG. 20B illustrates a perspective view of the fastening device of FIG. 20A in a second position.

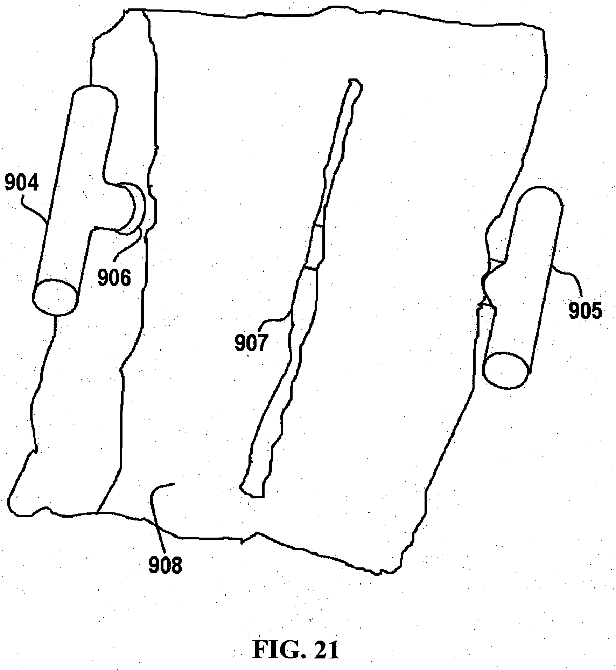

FIG. 21 illustrates a perspective view of the fastening device of FIG. 20A in an installed position.

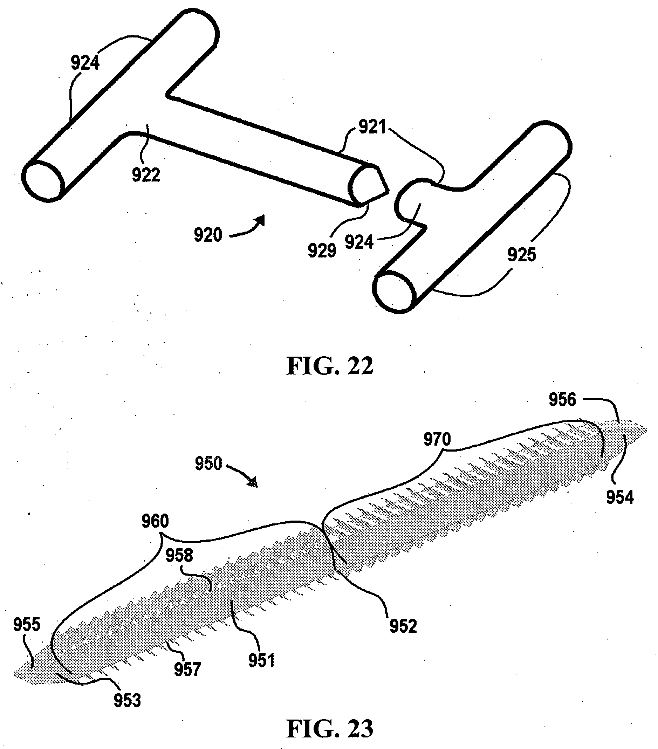

FIG. 22 illustrates a perspective view of an exemplary fastening device not forming part of the present disclosure.
FIG. 23 illustrates a perspective view of an exemplary fastening device not forming part of the present disclosure.

### Detailed Description of Exemplary Embodiments

Embodiments of the present disclosure provide a system for closing or approximating tissues, particularly mucosal tissues. The system can be particularly useful in surgeries and procedures involving the mucosal surfaces of the head and neck such as the oronasopharynx. Traditionally, placing sutures in mucosal tissues has been difficult given the propensity of the sutures to tear through the mucosal tissue. This is particularly problematic when tension is placed on the suture. The placement of sutures in the oronasopharynx, especially for procedures such as UPPP, UPF, or tonsillectomy, is especially time-consuming and difficult given the propensity of mucosa to tear and space constraints in this area. Embodiments of the present invention provide a specially designed fastening device for use in mucosal tissues that is useful in surgeries of the head and neck.

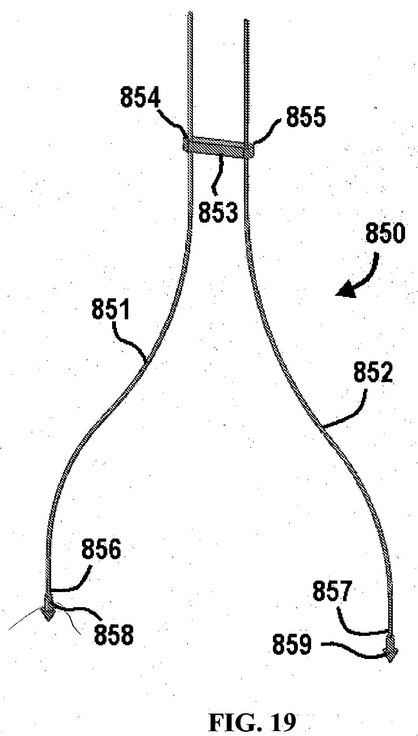

Referring now to FIG. 19, an exemplary embodiment of a fastening device 850 comprises of a first suture 851 and a second suture 852 coupled by a coupling member 853. First suture 851 comprises a first end 856 and second suture 852 comprises a first end 857. In this embodiment, a first fastening member 858 is coupled to first end 856 and a second fastening member 859 is coupled to first end 857. In the embodiment shown, first and second fastening members 858 and 859 each comprise a sharp pointed end to assist in penetrating tissue.

In the illustrated embodiment, coupling member 853 comprises a first aperture 854 and a second aperture 855. First suture 851 extends through first aperture 853 and second suture 852 extends through second aperture 854. During use, fastening member 858 can be used to pierce one side of an opening in tissue while the fastening member 859 can be used to pierce the opposite side of the opening in the tissue. Once fastening members 858 and 859 are anchored in opposite ends of the tissue opening, the coupling member 853 can be drawn toward fastening members 858 and 859. This can bring the tissue closer together to assist in closing the opening and expediting healing of the tissue. A knot (not shown) can be tied in each suture 851, 852 so that coupling member 853 is positioned between the knots and the fastening members 858, 859. First and second sutures 851, 852 can be tied together in a knot (not shown) so that coupling member 853 is positioned between the knot and the first and second fastening members 858, 859.

### Equivalents and Scope

The foregoing has been a description of certain non-limiting preferred embodiments of the invention. Those skilled in the art will recognize, or be able to ascertain using no more than routine experimentation, many equivalents to the specific embodiments of the invention described herein. Those of ordinary skill in the art will appreciate that various changes and modifications to this description may be made without departing from the scope of the present invention, as defined in the following claims.

In the claims articles such as "a", "an", and "the" may mean one or more than one unless indicated to the contrary or otherwise evident from the context. Claims or descriptions that include "or" between one or more members of a group are considered satisfied if one, more than one, or all of the group members are present in, employed in, or otherwise relevant to a given product or process unless indicated to the contrary or otherwise evident from the context. The invention includes embodiments in which exactly one member of the group is present in, employed in, or otherwise relevant to a given product or process. The invention also includes embodiments in which more than one, or all of the group members are present in, employed in, or otherwise relevant to a given product or process. Furthermore, it is to be understood that embodiments of the invention encompasses all variations, combinations, and permutations in which one or more limitations, elements, clauses, descriptive terms, *etc.,* from one or more of the claims or from relevant portions of the description is introduced into another claim. For example, any claim that is dependent on another claim can be modified to include one or more limitations found in any other claim that is dependent on the same base claim.

Where elements are presented as lists, *e.g.,* in Markush group format, it is to be understood that each subgroup of the elements is also disclosed, and any element(s) can be removed from the group. It is also noted that the term "comprising" is intended to be open and permits the inclusion of additional elements. It should be understood that, in general, where the invention, or aspects of the invention, is/are referred to as comprising particular elements, features, *etc*., certain embodiments of the invention or aspects of the invention consist, or consist essentially of, such elements, features *etc*. For purposes of simplicity those embodiments have not been specifically set forth *in haec verba* herein. Thus for each embodiment of the invention that comprises one or more elements, features, *etc.,* the invention also provides embodiments that consist or consist essentially of those elements, features *etc.*

Where ranges are given, endpoints are included. Furthermore, it is to be understood that unless otherwise indicated or otherwise evident from the context and/or the understanding of one of ordinary skill in the art, values that are expressed as ranges can assume any specific value within the stated ranges in different embodiments of the invention, to the tenth of the unit of the lower limit of the range, unless the context clearly dictates otherwise. It is also to be understood that unless otherwise indicated or otherwise evident from the context and/or the understanding of one of ordinary skill in the art, values expressed as ranges can assume any subrange within the given range, wherein the endpoints of the subrange are expressed to the same degree of accuracy as the tenth of the unit of the lower limit of the range.

In addition, it is to be understood that any particular embodiment of the present invention may be explicitly excluded from any one or more of the claims. Any embodiment, element, feature, application, or aspect of the compositions and/or methods of the invention can be excluded from any one or more claims. For example, in certain embodiments of the invention the biologically active agent is not an antiproliferative agent. For purposes of brevity, all of the embodiments in which one or more elements, features, purposes, or aspects is excluded are not set forth explicitly herein.

## Claims

1. A fastening device for fastening tissue, the fastening device comprising:
a first suture (851) comprising a first fastening member (858);
a second suture (852) comprising a second fastening member (859), and
a coupling member (853) coupling the first suture to the second suture; **characterised in that**; the first and second sutures are tied together in a knot; and the coupling member (853) is positioned between the knot and the first and second fastening members, wherein the coupling member is configured to slide relative to the first and second suture.

2. The fastening device of claim 1 wherein the coupling member (853) comprises a first aperture (854) and a second aperture (855), and wherein the first suture (851) extends through the first aperture and the second suture (852) extends through the second aperture.

3. The fastening device of claim 1 wherein the first fastening member (858) is coupled to a first end (856) of the first suture (851) and wherein the second fastening member (859) is coupled to a first end (857) of the second suture (852).

4. The fastening device of claim 1 wherein the first fastening member (858) and the second fastening member (859) each comprise a pointed end configured to penetrate tissue.

5. The fastening device of claim 1, wherein the fastening device comprises an elastomeric material.

6. The fastening device of claim 5 wherein the elastomeric material is a polyhydroxyalkanoate material.

7. The fastening device of claim 5 wherein the elastomeric material is combined with a crystalline or amorphous polymer.

8. The fastening device of claim 5 wherein the fastening device has an elasticity equivalent to that of mucosal tissue.

9. The fastening device of claim 1, wherein the fastening device comprises a shape memory material.

## Patentansprüche

1. Befestigungsvorrichtung zum Befestigen von Gewebe, wobei die Befestigungsvorrichtung Folgendes umfasst: eine erste Naht (851), die ein erstes Befestigungselement (858) umfasst;
eine zweite Naht (852), die ein zweites Befestigungselement (859) umfasst, und
ein Kopplungselement (853), das die erste Naht an die zweite Naht koppelt; **dadurch gekennzeichnet, dass**; die erste und zweite Naht miteinander verknotet sind; und das Kopplungselement (853) zwischen dem Knoten und dem ersten und zweiten Befestigungselement angeordnet ist, wobei das Kopplungselement dafür konfiguriert ist, dass es relativ zur ersten und zweiten Naht gleitet.

2. Befestigungsvorrichtung nach Anspruch 1, wobei das Kopplungselement (853) eine erste Öffnung (854) und eine zweite Öffnung (855) umfasst und wobei die erste Naht (851) sich durch die erste Öffnung und die zweite Naht (852) sich durch die zweite Öffnung erstreckt.

3. Befestigungsvorrichtung nach Anspruch 1, wobei das erste Befestigungselement (858) an ein erstes Ende (856) der ersten Naht (851) gekoppelt ist und wobei das zweite Befestigungselement (859) an ein erstes Ende (857) der zweiten Naht (852) gekoppelt ist.

4. Befestigungsvorrichtung nach Anspruch 1, wobei das erste Befestigungselement (858) und das zweite Befestigungselement (859) jeweils ein spitzes Ende umfassen, das für das Durchdringen von Gewebe konfiguriert ist.

5. Befestigungsvorrichtung nach Anspruch 1, wobei die Befestigungsvorrichtung ein Elastomermaterial umfasst.

6. Befestigungsvorrichtung nach Anspruch 5, wobei das Elastomermaterial ein Polyhydroxyalkanoatmaterial ist.

7. Befestigungsvorrichtung nach Anspruch 5, wobei das Elastomermaterial mit einem kristallinen oder amorphen Polymer kombiniert ist.

8. Befestigungsvorrichtung nach Anspruch 5, wobei die Befestigungsvorrichtung eine Elastizität aufweist, die äquivalent zu der von Schleimhautgewebe ist.

9. Befestigungsvorrichtung nach Anspruch 1, wobei die Befestigungsvorrichtung ein Formgedächtnismaterial umfasst.

## Revendications

1. Un dispositif de fixation pour la fixation de tissus, le dispositif de fixation comprenant :
une première suture (851) comprenant un premier élément de fixation (858) ;
une deuxième suture (852) comprenant un deuxième élément de fixation (859), et
un élément d'accouplement (853) accouplant la première suture avec la deuxième suture ; **caractérisé en ce que** : les première et deuxième sutures sont attachées l'une à l'autre en formant un noeud ; et l'élément d'accouplement (853) est positionné entre le noeud et les premier et deuxième éléments de fixation, l'élément d'accouplement étant configuré pour coulisser relativement à la première et la deuxième suture.

2. Le dispositif de fixation selon la revendication 1, l'élément d'accouplement (853) comprenant une première ouverture (854) et une deuxième ouverture (855), et la première suture (851) s'étendant à travers la première ouverture (854) et la deuxième suture (852) s'étendant à travers la deuxième ouverture.

3. Le dispositif de fixation selon la revendication 1, le premier élément de fixation (858) étant accouplé avec une première extrémité (856) de la première suture (851), et le deuxième élément de fixation (859) étant accouplé avec une première extrémité (857) de la deuxième suture (852).

4. Le dispositif de fixation selon la revendication 1, le premier élément de fixation (858) et le deuxième élément de fixation (859) comprenant chacun une extrémité pointue configurée pour pénétrer dans des tissus.

5. Le dispositif de fixation selon la revendication 1, le dispositif de fixation étant composé d'une matière élastomère.

6. Le dispositif de fixation selon la revendication 5, la matière élastomère étant une matière de polyhydroxyalcanoate.

7. Le dispositif de fixation selon la revendication 5, la matière élastomère étant alliée à un polymère cristallin ou amorphe.

8. Le dispositif de fixation selon la revendication 5, le dispositif de fixation selon la revendication 5, présentant une élasticité équivalente à celle d'un tissu muqueux.

9. Le dispositif de fixation selon la revendication 1, le dispositif de fixation comprenant un matériau à mémoire de forme.
